# EUROPEAN PATENT APPLICATION

(11) **EP 4 850 667 A1**
(43) Date of publication of application: **09.12.2026**
(21) Application number: 25748928.6
(22) Date of filing: 31.01.2025
(51) Int. Cl.: A61K 31/198, A61K 9/08, A61K 31/70, A61K 47/02, A61K 47/12, A61P 7/08

(54) **INFUSION PREPARATION AND PRODUCTION METHOD FOR SAME**

(30) Priority: 02.02.2024 JP 2024015054
(71) Applicant: Otsuka Pharmaceutical Factory, Inc., Naruto-shi, Tokushima 772-8601 (JP)
(72) Inventor: MORI, Hidetoshi, Naruto-shi, Tokushima 772-8601 (JP); NOMURA, Yoshiki, Naruto-shi, Tokushima 772-8601 (JP); KIUCHI, Yasuhiro, Naruto-shi, Tokushima 772-8601 (JP); SONOKI, Hiroyuki, Naruto-shi, Tokushima 772-8601 (JP)
(74) Representative: Hoffmann Eitle
(86) International application number: PCT/JP2025/003127
(87) International publication number: WO 2025/164749

(57) **Abstract**

The invention provides a two-chamber infusion preparation that is less likely to form a precipitate even upon a compositional change caused by the addition of a calcium ion correction solution. Provided is an infusion preparation for peripheral intravenous administration, comprising two chambers separated by a partition that is configured to allow fluid communication therebetween, the two chambers comprising a first chamber containing a first-chamber infusion containing an amino acid, and a second chamber containing a second-chamber infusion containing a sugar, wherein a mixed solution of the first-chamber infusion and the second-chamber infusion (A) has a pH of **6.4 or** less, and (B) contains an organic acid in a total amount of 3 mEq/L or more.

## Description

### Technical Field

The present invention relates to a two-chamber infusion preparation containing at least an amino acid and a sugar, the mixed solution of which is intended to be further mixed with a calcium ion correction solution, and to a method for producing the two-chamber infusion preparation.

### Background Art

When a correction solution having a specific electrolyte composition is required, an electrolyte correction solution may be mixed with another infusion preparation as needed depending on the individual case so that an appropriate infusion solution can be administered (PTL 1).

It has been reported that mixing Ca correction solutions, Mg correction solutions, and the like with other preparations may cause compositional changes and lead to precipitation, and that thus caution is required.

### Citation List

### Patent Literature

PTL 1: JP2011-153083A

### Summary of Invention

### Technical Problem

Many infusion preparations for peripheral parenteral nutrition are designed on the assumption that a correction solution will be added to the electrolyte solution of the infusion preparation. Normally, when a correction solution is added within an appropriate range, the precipitation stated above does not occur and no problem arises. On the other hand, it is known that when a correction solution is added to an electrolyte solution while an infusion bag is folded or suspended, adequate mixing cannot be performed, and the precipitation may occur, resulting in problems.

An object of the present invention is to provide a two-chamber infusion preparation that is less likely to form a precipitate even upon a compositional change caused by the addition of a calcium ion correction solution.

### Solution to Problem

The present inventors have conducted their own research and found that a two-chamber infusion preparation in which the mixed solution is adjusted to have a pH of 6.4 or less and to contain an organic acid in a total amount of 3 mEq/L or more is less likely to form a precipitate when mixed with a calcium ion correction solution.

Specifically, the present invention has been completed through further improvements and includes the embodiments stated in the following items.

### Item 1.

An infusion preparation for peripheral intravenous administration, comprising:
two chambers separated by a partition that is configured to allow fluid communication therebetween, the two chambers comprising:
a first chamber containing a first-chamber infusion containing an amino acid; and
a second chamber containing a second-chamber infusion containing a sugar,
wherein a mixed solution of the first-chamber infusion and the second-chamber infusion:
   (A) has a pH of 6.4 or less; and
   (B) contains an organic acid in a total amount of 3 mEq/L or more.

### Item 2.

The infusion preparation for peripheral intravenous administration according to Item 1, wherein the organic acid has a carboxy group.

### Item 3.

The infusion preparation for peripheral intravenous administration according to Item 1 or 2, wherein the organic acid is a hydroxycarboxylic acid or acetic acid.

### Item 4.

The infusion preparation for peripheral intravenous administration according to any one of Items 1 to 3, wherein the mixed solution has a pH of 6.0 to 6.4.

### Item 5.

The infusion preparation for peripheral intravenous administration according to any one of Items 1 to 4, wherein the mixed solution contains at least one selected from the group consisting of citric acid, lactic acid, and acetic acid in a total amount of 55 mEq/L or more, or contains one or more other organic acids in a total amount of 3 mEq/L or more.

### Item 6.

The infusion preparation for peripheral intravenous administration according to any one of Items 1 to 5, wherein the organic acid is gluconic acid and/or citric acid.

### Item 7.

The infusion preparation for peripheral intravenous administration according to any one of Item 1 to 6, wherein the liquid obtained by removing the organic acid(s) in a total amount of 3 mEq/L or more from the mixed solution has a pH of 6.7 or more.

### Item 8.

The infusion preparation for peripheral intravenous administration according to any one of Items 1 to 7, wherein the pH of the first-chamber infusion is 6.55 or less.

### Item 9.

A mixed solution of the first-chamber infusion and the second-chamber infusion according to any one of Items 1 to 8, obtained by allowing fluid communication through the partition.

### Item 10.

The infusion preparation for peripheral intravenous administration according to any one of Items 1 to 8, for use in mixing the mixed solution with a calcium ion correction solution.

### Item 11.

The infusion preparation for peripheral intravenous administration according to Item 10, wherein a ratio X/Y of a calcium ion content X (mEq/L) in the calcium ion correction solution to an organic acid content Y (mEq/L) in the mixed solution is 0.1 to 10.3.

### Item 12.

A method for producing an infusion preparation for peripheral intravenous administration,
the infusion preparation for peripheral intravenous administration comprising two chambers separated by a partition that is configured to allow fluid communication therebetween, the two chambers comprising:
   a first chamber containing a first-chamber infusion containing an amino acid; and
   a second chamber containing a second-chamber infusion containing a sugar,
wherein a mixed solution of the first-chamber infusion and the second-chamber infusion:
   (A) has a pH of 6.4 or less; and
   (B) contains an organic acid in a total amount of 3 mEq/L or more,
the method comprising:
adding an organic acid in a total amount of 3 mEq/L or more to the first-chamber infusion and/or the second-chamber infusion, wherein, before the addition, the mixed solution of the first-chamber infusion and the second-chamber infusion has a pH of 6.7 or more, thereby changing a composition of the first-chamber infusion and/or the second-chamber infusion so that the pH of the mixed solution is 6.4 or less.

### Advantageous Effects of Invention

The infusion preparation of the present invention has an effect of suppressing precipitation even upon a compositional change when the mixed solution is mixed with a calcium ion correction solution.

### Description of Embodiments

The present invention will be described below in further detail.

The present invention relates to an infusion preparation comprising two chambers separated by a partition that is configured to allow fluid communication therebetween, the two chambers comprising a first chamber containing a first-chamber infusion containing an amino acid, and a second chamber containing a second-chamber infusion containing a sugar.

In the infusion preparation of the present invention, the mixed solution of the first-chamber infusion and the second-chamber infusion:
(A) has a pH of 6.4 or less; and
(B) contains an organic acid in a total amount of 3 mEq/L or more. Due to this feature, the infusion preparation of the present invention has an effect of suppressing precipitation even upon a compositional change when the mixed solution is mixed with a calcium ion correction solution.

### First-chamber Infusion

The first-chamber infusion used in the present invention contains an amino acid. The first-chamber infusion contains an amino acid composition comprising at least an essential amino acid. The amino acid is contained at a concentration of 50 to 150 g/L, and preferably 80 to 120 g/L, in terms of free amino acids. Each amino acid used is preferably a pure crystalline amino acid, as in general-purpose amino acid infusion solutions. Although these amino acids are typically used in the form of free amino acids, they are not necessarily limited to the free amino acid form, and may also be used in the form of a pharmacologically acceptable salt, an ester, an N-acyl derivative, a salt of two amino acids, or a peptide. In particular, L-cysteine is preferably incorporated in the form of an N-acetyl derivative from the viewpoint of stability. The fluid volume of the first-chamber infusion to be contained in an infusion container is preferably 100 to 500 mL. The solvent for the first-chamber infusion is typically distilled water for injection.

Preferred amino acid compositions may include in terms of free amino acids, 5 to 20 (g/L) of L-leucine, 3 to 15 (g/L) of L-isoleucine, 3 to 15 (g/L) of L-valine, 3 to 15 (g/L) of L-lysine, 1.2 to 10 (g/L) of L-threonine, 0.3 to 5 (g/L) of L-tryptophan, 0.6 to 8 (g/L) of L-methionine, 1.8 to 15 (g/L) of L-phenylalanine, 0.1 to 3 (g/L) of L-cysteine, 0.06 to 2 (g/L) of L-tyrosine, 3 to 15 (g/L) of L-arginine, 1.2 to 10 (g/L) of L-histidine, 3 to 15 (g/L) of L-alanine, 1.2 to 10 (g/L) of L-proline, 0.6 to 7 (g/L) of L-serine, 1.2 to 10 (g/L) of glycine, 0.12 to 3 (g/L) of L-aspartic acid, and 0.12 to 3 (g/L) of L-glutamic acid.

The first-chamber infusion is adjusted to a pH of 6.57 or less, preferably 6.57 to 6.06, more preferably 6.46 to 6.06, by adding a small amount of a pH adjuster, as necessary. The pH adjuster is preferably an organic acid. When the pH is 6.55 or less, the pH after mixing can be easily maintained within the optimum range described below.

### Second-chamber Infusion

In the present invention, the second-chamber infusion contains a sugar. Specifically, for example, the second-chamber infusion may have a basic composition comprising glucose, a strong electrolyte, and vitamin B1, and may be free of sulfites in order to stabilize the vitamin B1.

The glucose is used at a concentration of 80 to 200 g/L, and preferably 80 to 150 g/L. In this glucose solution, the concentration of carboxylic acids and salts thereof is more preferably maintained at 0 to 5 mEq/L from the viewpoint of stabilizing vitamin B1. In order to minimize buffering capacity, it is preferable that the pH of the glucose solution is adjusted with a mineral acid, such as hydrochloric acid, and it is further preferable that all of the electrolytes to be contained are strong electrolytes. The pH of the second-chamber infusion is in the range of 3 to 5, and preferably 3.5 to 4.5. When the pH is less than 3, the stability of glucose is insufficient although the stability of vitamin B1 itself is excellent. On the other hand, when the pH exceeds 5, vitamin B1 becomes less stable.

The fluid volume of the glucose solution is preferably 200 to 1000 mL. The solvent for the glucose solution is typically distilled water for injection. In order to further improve the stability of vitamin B1 and also to facilitate adjustment of the pH of the mixed solution described below to 6 to 7.5, the titratable acidity of the glucose solution is preferably 1 or less, more preferably 0.5 or less, and still more preferably 0.1 or less.

In addition to glucose, one or more reducing sugars, such as fructose and maltose, may optionally be added in appropriate amounts, or one or more non-reducing sugars, such as sorbitol and glycerin, may optionally be added in appropriate amounts.

### Vitamin B1

Vitamin B1 is preferably added as thiamine to the second-chamber infusion at a concentration of 1 to 10 mg/L, and preferably 2 to 5 mg/L, and is suitably added in an absolute amount of 0.5 to 8 mg. As vitamin B1 (thiamine), thiamine hydrochloride, thiamine nitrate, prosultiamine, octotiamine, and the like may be used.

### Electrolyte

### (a) Potassium

Potassium is preferably added in divided portions to the second-chamber infusion and the first-chamber infusion. The potassium concentration is preferably 10 to 20 mEq/L in the second-chamber infusion and 20 to 40 mEq/L in the first-chamber infusion. The potassium is preferably incorporated in the first-chamber infusion and the second-chamber infusion in a total amount of 5 to 30 mEq.

The potassium source added to the second-chamber infusion is preferably a strong electrolyte, such as potassium chloride or potassium sulfate. In particular, potassium chloride is more preferred because it is widely used. On the other hand, the potassium source added to the first-chamber infusion may be a compound similar to those used for general-purpose electrolyte infusions and the like, and examples include potassium chloride, potassium acetate, potassium citrate, potassium dihydrogen phosphate, dipotassium hydrogen phosphate, potassium glycerophosphate, potassium sulfate, and potassium lactate. Of these, phosphates, such as potassium dihydrogen phosphate, dipotassium hydrogen phosphate, and potassium glycerophosphate, are preferred because they also serve as a phosphorus source. These potassium sources may be in the form of hydrates.

### (b) Calcium

Calcium is preferably added only to the second-chamber infusion. If calcium is added to the first-chamber infusion, it reacts with phosphate to form a precipitate. To avoid this, calcium is preferably kept separate. As the calcium source, it is preferable to use calcium chloride, which is a strong electrolyte. Calcium is preferably present in the second-chamber infusion at a concentration of 2 to 10 mEq/L.

### (c) Sodium

Sodium may be added to the first-chamber infusion or the second-chamber infusion, or both. Since it is preferable to use potassium and calcium as chlorides, it is preferable not to use sodium chloride as the sodium source in order to avoid hyperchloremic acidosis.

When a sodium salt having buffering capacity, such as sodium acetate, sodium citrate, sodium dihydrogen phosphate, disodium hydrogen phosphate, or sodium lactate, is used, the addition of sodium is preferably made to the first-chamber infusion in order to also satisfy the requirement for the titratable acidity of the second-chamber infusion. Sodium is preferably present in the first-chamber infusion at a concentration of 80 to 150 mEq/L.

In order to prevent precipitation of phosphorus with calcium or magnesium after mixing, it is preferable to use sodium citrate as part of the sodium source.

### (d) Other Electrolytes

(i) Examples of magnesium sources include magnesium sulfate, magnesium chloride, and magnesium acetate. Of these, magnesium sulfate and magnesium chloride may be added to the second-chamber infusion as strong electrolytes.
(ii) Examples of phosphorus sources include potassium dihydrogen phosphate, dipotassium hydrogen phosphate, potassium glycerophosphate, sodium dihydrogen phosphate, disodium hydrogen phosphate, and sodium glycerophosphate. These phosphorus compounds may be added to the first-chamber infusion.
(iii) Examples of zinc sources include zinc sulfate and zinc chloride. These zinc compounds may be added to the second-chamber infusion.

Hydrates may also be used as the electrolyte sources (i) to (iii); however, electrolytes having buffering capacity must be added to the first-chamber infusion. The concentration of each electrolyte is preferably such that the second-chamber infusion contains magnesium at about 2 to 10 mEq/L and zinc at about 2 to 10 mmol/L, and that the first-chamber infusion contains phosphorus at about 10 to 20 mmol/L.

### Additives and Other Components

Additives, such as stabilizers, may be added to the infusion preparation of the present invention, as necessary. Sulfites, such as sodium bisulfite, which is a typical stabilizer, are suitably added to the first-chamber infusion. Other agents, including, for example, various vitamins and trace elements (minerals), may also be optionally added to the infusion preparation of the present invention, as necessary.

### Organic Acid

The organic acid is not particularly limited, and any organic acid usable as components of infusion preparations may be broadly selected as long as the effects of the present invention are exerted. The organic acids may be used singly or in a combination of two or more.

The infusion preparation of the present invention is characterized in that the mixed solution contains an organic acid in a total amount of 3 mEq/L or more. Preferably, in the infusion preparation of the present invention, the mixed solution further contains, in addition to the organic acid contained in the electrolyte, one or more of the same and/or different organic acids in a total amount of 3 mEq/L or more.

Examples of the organic acid include organic acids having a carboxy group.

Further specific examples of the organic acid include hydroxycarboxylic acids and acetic acid. Examples of hydroxycarboxylic acids include gluconic acid, citric acid, lactic acid, succinic acid, tartaric acid, and malic acid. The organic acid is preferably gluconic acid and/or citric acid from the viewpoint of the effect of suppressing precipitation.

The mixed solution preferably contains at least one selected from the group consisting of citric acid, lactic acid, and acetic acid in a total amount of 55 mEq/L or more, or contains one or more other organic acids in a total amount of 3 mEq/L or more.

The pH of the mixed solution is preferably 6.7 or more, and more preferably 6.75 or more, after removal of the organic acids in a total amount of 3 mEq/L or more.

### Mixed Solution

It is desirable that the concentration of the sugar in the mixed solution is set within a range of 50 to 100 g/L, and preferably 50 to 90 g/L.

It is desirable that the concentration of vitamin B1 in the mixed solution is set within a range of 0.5 to 8 mg/L, calculated as thiamine.

A preferred example of the concentrations of amino acids in the mixed solution is as follows: in terms of free amino acids, 2.5 to 20 g/L of L-leucine, 1.5 to 15 g/L of L-isoleucine, 1.5 to 15 g/L of L-valine, 1.5 to 15 g/L of L-lysine, 0.6 to 10 g/L of L-threonine, 0.15 to 5 g/L of L-tryptophan, 0.3 to 8 g/L of L-methionine, 0.85 to 15 g/L of L-phenylalanine, 0.03 to 3 g/L of L-cysteine, 0.03 to 2 g/L of L-tyrosine, 1.5 to 15 g/L of L-arginine, 0.6 to 10 g/L of L-histidine, 1.5 to 15 g/L of L-alanine, 0.6 to 10 g/L of L-proline, 0.3 to 7 g/L of L-serine, 0.6 to 10 g/L of glycine, 0.06 to 3 g/L of L-aspartic acid, and 0.06 to 3 g/L of L-glutamic acid.

It is desirable that the concentration of potassium in the mixed solution is set within a range of 5 to 30 mEq/L. It is also desirable that the concentration of potassium in the mixed solution is 16 mEq/L or more.

It is desirable that the concentration of calcium in the mixed solution is set within a range of 2 to 8 mEq/L. It is also desirable that the concentration of calcium in the mixed solution is 9 mEq/L or less.

It is desirable that the concentration of sodium in the mixed solution is set within a range of 20 to 50 mEq/L, and preferably 30 to 50 mEq/L.

It is desirable that the concentration of magnesium in the mixed solution is set within a range of 0.5 to 10 mEq/L, and preferably 1 to 7 mEq/L.

It is desirable that the concentration of phosphorus in the mixed solution is set within a range of 1 to 20 mmol/L, and preferably 5 to 10 mEq/L.

It is desirable that the concentration of zinc in the mixed solution is set within a range of 2 to 10 µmol/L.

The pH of the mixed solution is preferably 6.0 to 6.4, more preferably 6.1 to 6.3, and still more preferably 6.11 to 6.21. When the pH is 6.4 or less, an effect of suppressing precipitation is more readily exhibited even upon a compositional change when the mixed solution is mixed with a calcium ion correction solution. When the pH is 6.0 or more, an effect of facilitating the reduction of vascular pain during peripheral intravenous administration is exhibited.

The ratio X/Y of the calcium ion content X (mEq/L) in the calcium ion correction solution to the organic acid content Y (mEq/L) in the mixed solution is preferably 0.1 to 10.3. The closer the ratio X/Y is to zero, the less likely precipitation is to occur due to a compositional change caused by the addition of a calcium ion correction solution. According to the present disclosure, when the pH of the mixed solution is 6.4 and succinic acid is used as the organic acid, and when the organic acid concentration is 3.9 mEq/L, it has been confirmed that precipitation is suppressed even after addition of 40 meq/L of calcium ions. This indicates that precipitation caused by a compositional change can be suppressed insofar as the ratio X/Y is 10.3 or less.

### Infusion Container

The container in which the first-chamber infusion and the second-chamber infusion are placed is not particularly limited insofar as the container has two chambers that are intercommunicable. Examples include two-chamber containers (infusion bags) in which the chambers are separated by a partition wall that is configured to allow fluid communication therebetween, such as ones in which an easily peelable seal serves as the partition wall (Japanese Unexamined Patent Publication No. H2-4671, Japanese Unexamined Utility Model Publication No. H5-5138, and the like), ones in which a partition wall is formed by clipping the space between the chambers (Japanese Unexamined Patent Publication No. S63-309263 and the like), and ones in which various communication means capable of opening the partition wall are provided to the partition wall (Japanese Examined Patent Publication No. S63-20550 and the like). Of these, an infusion bag in which an easily peelable seal (easy-peel seal) serves as the partition wall is preferable because it is suitable for mass production and the chambers can be easily brought into communication. Further, various gaspermeable plastics commonly used for medical containers may be used as materials of the container. Examples include flexible plastics, such as polyethylene, polypropylene, polyvinyl chloride, crosslinked ethylene-vinyl acetate copolymer, ethylene-α-olefin copolymer, blends of such polymers, and laminates comprising such polymers.

The first- and second-chamber infusions can be put into and packaged in the container by a conventional method. For example, the chambers may be filled with the infusions under an inert gas atmosphere, sealed, and sterilized by heat.

Sterilization by heat can be performed by a known method, such as high-pressure steam sterilization or hot water shower sterilization. If necessary, sterilization by heat can be carried out in an inert gas atmosphere, such as carbon dioxide or nitrogen. The infusion preparation of the present invention does not produce unwanted insoluble matter even when sterilization is performed in high-pressure steam at 116 to 121°C.

Further, the first- and second-chamber infusions contained in the container are preferably packaged together with a deoxidant in an oxygen barrier exterior bag in order to reliably prevent deterioration, oxidation, and the like. In particular, when an infusion bag in which an easily peelable seal serves as the partition wall is used as the container, the infusion bag is preferably packaged in such a manner that the infusion bag is folded, for example, in half, at the easily peelable seal portion to prevent the partition wall from being communicably opened by external pressure. Further, for example, filling and packaging may optionally be performed under an inert gas atmosphere.

Commonly used films, sheets, and the like formed from various materials can be used as materials for the oxygen barrier exterior bag suitable for the package. Specific examples include ethylene-vinyl alcohol copolymer, polyvinylidene chloride, polyacrylonitrile, polyvinyl alcohol, polyamide, polyester, aluminum foil, and metallized film, as well as films and sheets formed from materials comprising at least one of these materials.

Usable deoxidants include various known deoxidants, such as those comprising, as an active ingredient, ferric hydroxide, ferric oxide, iron carbide, or other iron compounds, and those comprising low-molecular-weight phenol and activated carbon. Examples of product names of typical commercial products include Ageless (Mitsubishi Gas Chemical Company, Inc.), Moduran (Nippon Kayaku Co., Ltd.), Secure (Nippon Soda Co., Ltd.), Tamotsu (Oji Kako Co., Ltd.), and Keepit (Dorency Co., Ltd.).

### Mode of Use of Infusion Preparation

The infusion preparation of the present invention is used for nutritional management of preoperative and postoperative patients, and is used for patients with mild hypoproteinemia or mild malnutrition due to inadequate oral intake or for patients in the postoperative stress phase. In particular, the infusion preparation is suitably used for nutritional management of patients having difficulty receiving oral nutritional support in the postoperative period or due to a digestive disorder and the like (preferably, patients who have undergone gastric resection surgery). The infusion preparation of the present invention is administered to a patient for 1 to 14 days after surgery, and preferably 1 to 3 days after surgery. The nutritional status of the patient can thereby be maintained in a healthy state. The dose and the dosing rate can be suitably determined in view of each patient's symptoms, age, and the like. In particular, when the infusion preparation of the present invention is used, the infusion preparation can maintain, by itself, the nutritional status of the patient in a healthy state for the duration of administration.

The infusion preparation of the present invention is administered into a peripheral vein. In other words, the infusion preparation of the present invention is an infusion preparation for peripheral intravenous administration. In general, administration of an infusion that has overly high osmotic pressure into a peripheral vein can cause vascular pain or phlebitis. However, there is no such risk when the infusion preparation of the present invention is used. Therefore, the effect of the infusion preparation of the present invention is suitably exerted when the infusion preparation is administered into a peripheral vein.

In particular, the infusion preparation of the present invention has an effect of suppressing precipitation even upon a compositional change when the mixed solution is mixed with a calcium ion correction solution. Therefore, the infusion preparation of the present invention is preferably used for such applications.

When the mixed solution is mixed with a calcium ion correction solution, the calcium ion correction solution preferably contains 360 to 1000 mEq/L of calcium ions. In this case, when calcium chloride hydrate is contained as a component, its amount is preferably 0.5 g to 1.5 g, and when calcium gluconate hydrate is contained as a component, its amount is preferably 0.4 g to 0.9 g. It is also desirable that the concentration of calcium ions is 1.8 to 40 mEq/L after the calcium ion correction solution is mixed with the mixed solution of the present invention.

When the mixed solution is mixed with a calcium ion correction solution, the infusion preparation of the present invention preferably does not form a precipitate within 0.5 hours after mixing, more preferably within 1 hour after mixing, still more preferably within 3 hours after mixing, even more preferably within 6 hours after mixing, still even more preferably within 15 hours after mixing, and most preferably within 24 hours after mixing. In the present invention, the presence or absence of precipitation can be confirmed visually.

### Examples

The present invention will be described below in detail; however, the present invention is not limited to the following Examples.

Table 1 shows the composition of the first-chamber infusion before the addition of an acid. Table 2 shows the composition of the second-chamber infusion before the addition of an acid. Table 3 shows the composition after mixing the first-chamber infusion and second-chamber infusion. Using the two-chamber infusion preparation having the composition shown in Table 3 as a basis, various infusion preparations were prepared by appropriately adding organic acids as shown in Tables 4 to 8. Tables 4 to 8 show only the electrolytes and organic acids in the compositions. Tables 4 to 8 also show the pH of the infusions and the mixed solution.

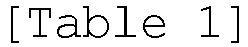

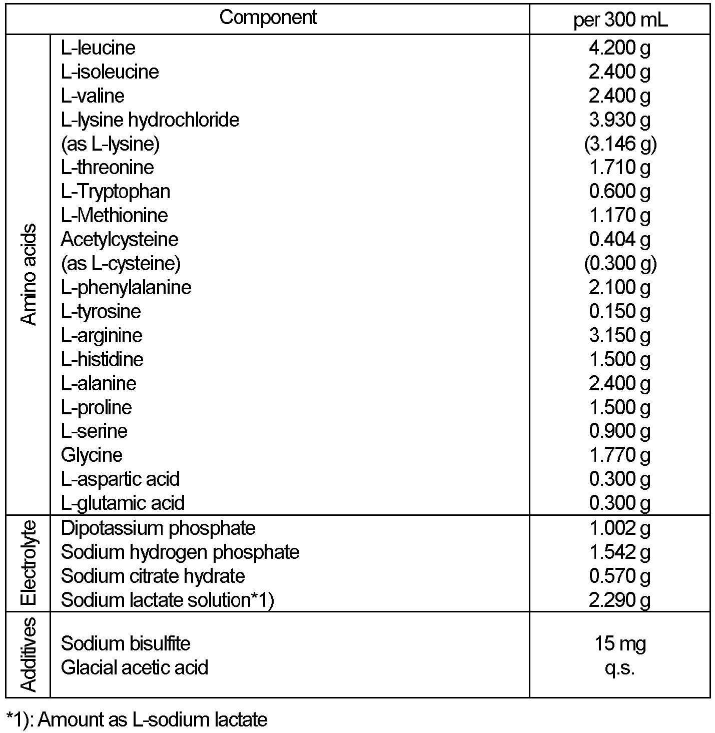

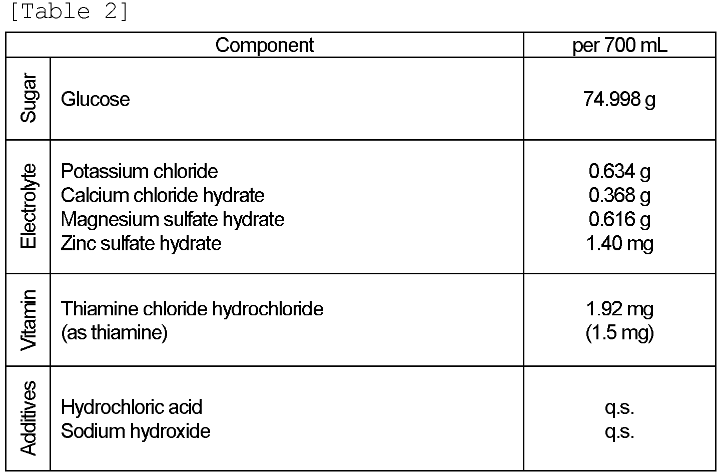

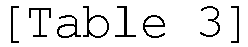

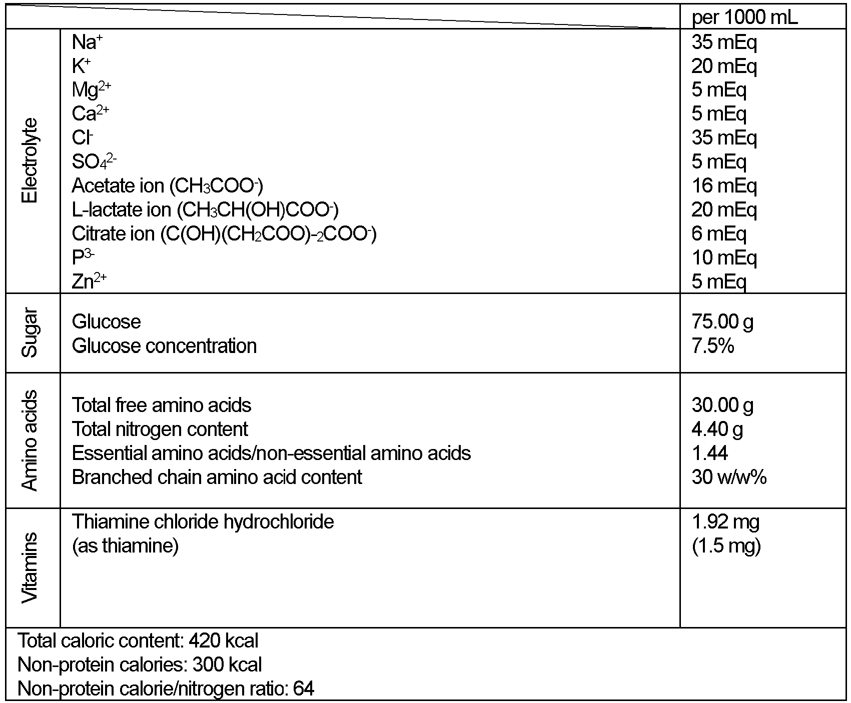

**[Table 4]**

| | | Original composition | After addition of acetic acid | |
|---|---|---|---|---|
| | | per 1000 mL | Minimum to maximum | |
| | Na⁺ | 35 mEq | - | - |
| | K⁺ | 20 mEq | - | - |
| | Mg²⁺ | 5 mEq | - | - |
| | Ca²⁺ | 5 mEq | - | - |
| | Cl⁻ | 35 mEq | - | - |
| | SO₄²⁻ | 5 mEq | - | - |
| Electrolyte | Acetate⁻ | 16 mEq | 20.3 to 25.7 mEq | |
| | L-Lactate⁻ | 20 mEq | - | - |
| | Citrate³⁻ | 6 mEq | - | - |
| | P | 10 mmol | - | - |
| | Zn | 5 µ mol | - | - |
| | Gluconate⁻ | 0 mEq | - | - |
| | Succinate²⁻ | 0 mEq | - | - |
| pH | First-chamber infusion | 6.3 to 7.3 | 6.12 to 6.54 | |
| | Second-chamber infusion | 3.5 to 4.5 | - | - |
| | When mixed | Approx. 6.7 | 6.00 to 6.40 | |

**[Table 5]**

| | | Original composition | After addition of gluconic acid | |
|---|---|---|---|---|
| | | per 1000 mL | Minimum to maximum | |
| | Na⁺ | 35 mEq | - | - |
| | K⁺ | 20 mEq | - | - |
| | Mg²⁺ | 5 mEq | - | - |
| | Ca²⁺ | 5 mEq | - | - |
| | Cl⁻ | 35 mEq | - | - |
| | SO₄²⁻ | 5 mEq | - | - |
| Electrolyte | Acetate⁻ | 16 mEq | - | - |
| | L-Lactate⁻ | 20 mEq | - | - |
| | Citrate³⁻ | 6 mEq | - | - |
| | P | 10 mmol | - | - |
| | Zn | 5 µ mol | - | - |
| | Gluconate⁻ | 0 mEq | 6.8 to 15.2 mEq | |
| | Succinate²⁻ | 0 mEq | - | - |
| pH | First-chamber infusion | 6.3 to 7.3 | 6.06 to 6.50 | |
| | Second-chamber infusion | 3.5 to 4.5 | - | - |
| | When mixed | Approx. 6.7 | 6.00 to 6.41 | |

**[Table 6]**

| | | Original composition | After addition of lactic acid | |
|---|---|---|---|---|
| | | per 1000 mL | Minimum to maximum | |
| | Na⁺ | 35 mEq | - | - |
| | K⁺ | 20 mEq | - | - |
| | Mg²⁺ | 5 mEq | - | - |
| | Ca²⁺ | 5 mEq | - | - |
| | Cl⁻ | 35 mEq | - | - |
| | SO₄²⁻ | 5 mEq | - | - |
| Electrolyte | Acetate⁻ | 16 mEq | - | - |
| | L-Lactate⁻ | 20 mEq | 25.5 to 30.5 mEq | |
| | Citrate³⁻ | 6 mEq | - | - |
| | P | 10 mmol | - | - |
| | Zn | 5 µ mol | - | - |
| | Gluconate⁻ | 0 mEq | - | - |
| | Succinate²⁻ | 0 mEq | - | - |
| pH | First-chamber infusion | 6.3 to 7.3 | 6.22 to 6.52 | |
| | Second-chamber infusion | 3.5 to 4.5 | - | - |
| | When mixed | Approx. 6.7 | 6.01 to 6.40 | |

**[Table 7]**

| | | Original composition | After addition of citric acid | |
|---|---|---|---|---|
| | | per 1000 mL | Minimum to maximum | |
| | Na⁺ | 35 mEq | - | - |
| | K⁺ | 20 mEq | - | - |
| | Mg²⁺ | 5 mEq | - | - |
| | Ca²⁺ | 5 mEq | - | - |
| | Cl⁻ | 35 mEq | - | - |
| | SO₄²⁻ | 5 mEq | - | - |
| Electrolyte | Acetate⁻ | 16 mEq | - | - |
| | L-Lactate⁻ | 20 mEq | - | - |
| | Citrate³⁻ | 6 mEq | 10.3 to 15.9 mEq | |
| | P | 10 mmol | - | - |
| | Zn | 5 µ mol | - | - |
| | Gluconate⁻ | 0 mEq | - | - |
| | Succinate²- | 0 mEq | - | - |
| pH | First-chamber infusion | 6.3 to 7.3 | 6.14 to 6.53 | |
| | Second-chamber infusion | 3.5 to 4.5 | - | - |
| | When mixed | Approx. 6.7 | 6.01 to 6.41 | |

**[Table 8]**

| | | Original composition | After addition of succinic acid | |
|---|---|---|---|---|
| | | per 1000 mL | Minimum to maximum | |
| | Na⁺ | 35 mEq | - | - |
| | K⁺ | 20 mEq | - | - |
| | Mg²⁺ | 5 mEq | - | - |
| | Ca²⁺ | 5 mEq | - | - |
| | Cl⁻ | 35 mEq | - | - |
| | SO₄²⁻ | 5 mEq | - | - |
| Electrolyte | Acetate⁻ | 16 mEq | - | - |
| | L-Lactate⁻ | 20 mEq | - | - |
| | Citrate³⁻ | 6 mEq | - | - |
| | P | 10 mmol | - | - |
| | Zn | 5 µ mol | - | - |
| | Gluconate⁻ | 0 mEq | - | - |
| | Succinate²⁻ | 0 mEq | 3.9 to 9.4 mEq | |
| pH | First-chamber infusion | 6.3 to 7.3 | 6.15 to 6.57 | |
| | Second-chamber infusion | 3.5 to 4.5 | - | - |
| | When mixed | Approx. 6.7 | 6.01 to 6.40 | |

The effects of compositional changes caused by the addition of a calcium chloride correction solution to the mixed solution were tested as follows. Specifically, a case was assumed in which a single ampoule (20 mL) of a 1 mEq/mL calcium chloride correction solution was added to 500 mL of a mixed solution. This process was scaled down as described below and tested in a test tube.
(1) 150 mL of the first-chamber infusion and 350 mL of the second-chamber infusion were measured with a graduated cylinder and mixed together in a beaker.
(2) While a pH meter (model: HM-30R, Dkk-Toa Corporation) was inserted, several microliters of an organic acid was added to the solution of (1). The organic acid was added until the pH of the mixed solution reached 6.7.
(3) The resulting mixture was transferred to a 20 mL test tube.
(4) The organic acid was added to lower the pH by 0.1 from the pH obtained in step (3), and the process returned to the operation in step (3).
(5) The operations in steps (3) to (4) were repeated until the pH of the mixed solution in the beaker reached 6.0, thereby preparing test tubes, each containing 20 mL of the mixed solution having a pH of 6.7 to 6.0.
(6) To each test tube containing 20 mL of the solution, 0.8 mL of 1 mEq/L calcium chloride correction solution was added to the surface. The mixture was then mixed by pipetting 5 mL of the mixture three times.
(7) Each test tube was sealed with Parafilm to prevent evaporation of the solution.
(8) Time-course observation was performed, and the results were evaluated as follows: "○": colorless and transparent; "×": white turbidity.

The results are shown below. Comparative Examples in which hydrochloric acid, sulfuric acid, nitric acid, and phosphoric acid were added instead of an organic acid are also shown (Tables 9 to 17).

**[Table 9]**

| | | 0.5 mol/L sulfuric acid added | | | | | | | |
|---|---|---|---|---|---|---|---|---|---|
| | Amount of acid added (amount added for that pH only) | (mL) | 0.6 | 0.5 | 0.5 | 0.6 | 0.5 | 0.5 | 0.5 |
| | | 0.2 | | | | | | | |
| | pH of the mixed solution | 6.70 | 6.59 | 6.50 | 6.41 | 6.30 | 6.20 | 6.10 | 5.99 |
| | pH of the first-chamber infusion | 6.82 | 6.71 | 6.62 | 6.52 | 6.41 | 6.32 | 6.22 | 6.11 |

| | 0.5 mol/L | | | | | | | | |
|---|---|---|---|---|---|---|---|---|---|
| Amount of acid added etc. | | 0.5 mol/L sulfuric acid added | | | | | | | |
| | pH of the mixed solution | 6.70 | 6.59 | 6.50 | 6.41 | 6.30 | 6.20 | 6.10 | 5.99 |
| | pH of the first-chamber infusion | 6.82 | 6.71 | 6.62 | 6.52 | 6.41 | 6.32 | 6.22 | 6.11 |
| | Total amount of acid added (mL) (taking into account the amount of acid removed together when 20 mL of the original composition is withdrawn each time) | 0.2 | 0.8 | 1.3 | 1.7 | 2.2 | 2.6 | 3.0 | 3.3 |
| | Total amount of acid added (meq) (taking into account the amount of acid removed together when 20 mL of the original composition is withdrawn each time) | 0.1 | 0.4 | 0.6 | 0.9 | 1.1 | 1.3 | 1.5 | 1.7 |
| | Valency of acid | 2 | 2 | 2 | 2 | 2 | 2 | 2 | 2 |
| | Remaining amount of the original composition (mixed solution) (mL) | 500 | 480 | 460 | 440 | 420 | 400 | 380 | 360 |
| | Total amount of acid added (mEq/L) | 0.4 | 1.7 | 2.7 | 3.9 | 5.3 | 6.6 | 7.9 | 9.3 |
| Compositional change table (original composition: 500 mL + 1 ampoule (20 mL) of 1 meq/mL CaCl₂ correction solution) | Immediately after addition | × | × | × | × | ○ | ○ | ○ | ○ |
| | After 1 hour | × | × | × | × | ○ | ○ | ○ | ○ |
| | After 3 hours | × | × | × | × | × | ○ | ○ | ○ |
| | After 6 hours | × | × | × | × | × | ○ | ○ | ○ |
| | After 24 hours | × | × | × | × | × | × | ○ | ○ |

**[Table 10]**

| | | 0.5 mol/L nitric acid added | | | | | | | |
|---|---|---|---|---|---|---|---|---|---|
| | Amount of acid added (amount added for that pH only) | (mL) | 1.1 | 1.1 | 1.1 | 1.1 | 1.0 | 1.0 | 1.0 |
| | | 0.4 | | | | | | | |
| | pH of the mixed solution | 6.70 | 6.60 | 6.50 | 6.40 | 6.30 | 6.20 | 6.10 | 5.99 |
| | pH of the first-chamber infusion | 6.82 | 6.72 | 6.62 | 6.51 | 6.41 | 6.31 | 6.20 | 6.09 |

| | 0.5 mol/L | | | | | | | | |
|---|---|---|---|---|---|---|---|---|---|
| Amount of acid added etc. | | 0.5 mol/L nitric acid added | | | | | | | |
| | pH of the mixed solution | 6.70 | 6.60 | 6.50 | 6.40 | 6.30 | 6.20 | 6.10 | 5.99 |
| | pH of the first-chamber infusion | 6.82 | 6.72 | 6.62 | 6.51 | 6.41 | 6.31 | 6.20 | 6.09 |
| | Total amount of acid added (mL) (taking into account the amount of acid removed together when 20 mL of the original composition is withdrawn each time) | 0.4 | 1.5 | 2.5 | 3.5 | 4.5 | 5.2 | 6.0 | 6.7 |
| | Total amount of acid added (meq) (taking into account the amount of acid removed together when 20 mL of the original composition is withdrawn each time) | 0.2 | 0.7 | 1.3 | 1.8 | 2.2 | 2.6 | 3.0 | 3.3 |
| | Valency of acid | 1 | 1 | 1 | 1 | 1 | 1 | 1 | 1 |
| | Remaining amount of the original composition (mixed solution) (mL) | 500 | 480 | 460 | 440 | 420 | 400 | 380 | 360 |
| | Total amount of acid added (mEq/L) | 0.4 | 1.5 | 2.7 | 4.0 | 5.3 | 6.6 | 7.9 | 9.3 |
| Compositional change table (original composition: 500 mL + 1 ampoule (20 mL) of 1 meq/mL CaCl₂ correction solution) | Immediately after addition | × | × | × | × | ○ | ○ | ○ | ○ |
| | After 1 hour | × | × | × | × | ○ | ○ | ○ | ○ |
| | After 3 hours | × | × | × | × | × | ○ | ○ | ○ |
| | After 6 hours | × | × | × | × | × | ○ | ○ | ○ |
| | After 24 hours | × | × | × | × | × | × | ○ | ○ |

**[Table 11]**

| | | 0.5 mol/L nitric acid added | | | | | | | |
|---|---|---|---|---|---|---|---|---|---|
| | Amount of acid added (amount added for that pH only) | (mL) | 1.1 | 1.1 | 1.1 | 1.1 | 1.0 | 1.0 | 1.0 |
| | | 0.4 | | | | | | | |
| | pH of the mixed solution | 6.70 | 6.60 | 6.50 | 6.40 | 6.30 | 6.20 | 6.10 | 5.99 |
| | pH of the first-chamber infusion | 6.82 | 6.72 | 6.62 | 6.51 | 6.41 | 6.31 | 6.20 | 6.09 |

| | 0.5 mol/L | | | | | | | | |
|---|---|---|---|---|---|---|---|---|---|
| Amount of acid added etc. | | 0.5 mol/L nitric acid added | | | | | | | |
| | pH of the mixed solution | 6.70 | 6.60 | 6.50 | 6.40 | 6.30 | 6.20 | 6.10 | 5.99 |
| | pH of the first-chamber infusion | 6.82 | 6.72 | 6.62 | 6.51 | 6.41 | 6.31 | 6.20 | 6.09 |
| | Total amount of acid added (mL) (taking into account the amount of acid removed together when 20 mL of the original composition is withdrawn each time) | 0.4 | 1.5 | 2.5 | 3.5 | 4.5 | 5.2 | 6.0 | 6.7 |
| | Total amount of acid added (meq) (taking into account the amount of acid removed together when 20 mL of the original composition is withdrawn each time) | 0.2 | 0.7 | 1.3 | 1.8 | 2.2 | 2.6 | 3.0 | 3.3 |
| | Valency of acid | 1 | 1 | 1 | 1 | 1 | 1 | 1 | 1 |
| | Remaining amount of the original composition (mixed solution) (mL) | 500 | 480 | 460 | 440 | 420 | 400 | 380 | 360 |
| | Total amount of acid added (mEq/L) | 0.4 | 1.5 | 2.7 | 4.0 | 5.3 | 6.6 | 7.9 | 9.3 |
| Compositional change table (original composition: 500 mL + 1 ampoule (20 mL) of 1 meq/mL CaCl₂ correction solution) | Immediately after addition | × | × | × | × | ○ | ○ | ○ | ○ |
| | After 1 hour | × | × | × | × | ○ | ○ | ○ | ○ |
| | After 3 hours | × | × | × | × | × | ○ | ○ | ○ |
| | After 6 hours | × | × | × | × | × | ○ | ○ | ○ |
| | After 24 hours | × | × | × | × | × | × | ○ | ○ |

**[Table 12]**

| | | 0.5 mol/L phosphoric acid | | | | | | | |
|---|---|---|---|---|---|---|---|---|---|
| | Amount of acid added (amount added for that pH only) | (mL) | 0.8 | 0.8 | 0.9 | 1.0 | 0.9 | 1.0 | 1.0 |
| | | 0.3 | | | | | | | |
| | pH of the mixed solution | 6.70 | 6.59 | 6.50 | 6.40 | 6.30 | 6.20 | 6.10 | 6.00 |
| | pH of the first-chamber infusion | 6.82 | 6.71 | 6.60 | 6.50 | 6.38 | 6.28 | 6.17 | 6.06 |

| | 0.5 mol/L | | | | | | | | |
|---|---|---|---|---|---|---|---|---|---|
| Amount of acid added etc. | | 0.5 mol/L phosphoric acid | | | | | | | |
| | pH of the mixed solution | 6.70 | 6.59 | 6.50 | 6.40 | 6.30 | 6.20 | 6.10 | 6.00 |
| | pH of the first-chamber infusion | 6.82 | 6.71 | 6.60 | 6.50 | 6.38 | 6.28 | 6.17 | 6.06 |
| | Total amount of acid added (mL) (taking into account the amount of acid removed together when 20 mL of the original composition is withdrawn each time) | 0.3 | 1.1 | 1.8 | 2.7 | 3.5 | 4.3 | 5.1 | 5.8 |
| | Total amount of acid added (meq) (taking into account the amount of acid removed together when 20 mL of the original composition is withdrawn each time) | 0.2 | 0.5 | 0.9 | 1.3 | 1.8 | 2.1 | 2.5 | 2.9 |
| | Valency of acid | 1 | 1 | 1 | 1 | 1 | 1 | 1 | 1 |
| | Remaining amount of the original composition (mixed solution) (mL) | 500 | 480 | 460 | 440 | 420 | 400 | 380 | 360 |
| | Total amount of acid added (mEq/L) | 0.3 | 1.1 | 2.0 | 3.0 | 4.2 | 5.3 | 6.7 | 8.0 |
| Compositional change table (original composition: 500 mL + 1 ampoule (20 mL) of 1 meq/mL CaCl₂ correction solution) | Immediately after addition | × | × | × | × | × | ○ | ○ | ○ |
| | After 1 hour | × | × | × | × | × | × | ○ | ○ |
| | After 3 hours | × | × | × | × | × | × | ○ | ○ |
| | After 6 hours | × | × | × | × | × | × | ○ | ○ |
| | After 24 hours | × | × | × | × | × | × | × | ○ |

**[Table 13]**

| | | Unadjusted | Acetic acid added | | | | | | | |
|---|---|---|---|---|---|---|---|---|---|---|
| | Amount of acid added (amount added for that pH only) | 0 | (µL) | 30 | 35 | 35 | 30 | 35 | 30 | 25 |
| | | | 15 | | | | | | | |
| | pH of the mixed solution | 6.76 | 6.70 | 6.61 | 6.51 | 6.40 | 6.31 | 6.19 | 6.10 | 6.00 |
| | pH of the first-chamber infusion | 6.91 | 6.86 | 6.77 | 6.65 | 6.54 | 6.44 | 6.32 | 6.21 | 6.12 |

| | 1.75 mol/L | | | | | | | | | |
|---|---|---|---|---|---|---|---|---|---|---|
| Amount of acid added etc. | | Unadjusted | Acetic acid added | | | | | | | |
| | pH of the mixed solution | 6.76 | 6.70 | 6.61 | 6.51 | 6.40 | 6.31 | 6.19 | 6.10 | 6.00 |
| | pH of the first-chamber infusion | 6.91 | 6.86 | 6.77 | 6.65 | 6.54 | 6.44 | 6.32 | 6.21 | 6.12 |
| | Total amount of acid added (mL) (taking into account the amount of acid removed together when 20 mL of the original composition is withdrawn each time) | - | 0.0 | 0.0 | 0.1 | 0.1 | 0.1 | 0.2 | 0.2 | 0.2 |
| | Total amount of acid added (meq) (taking into account the amount of acid removed together when 20 mL of the original composition is withdrawn each time) | - | 0.3 | 0.8 | 1.4 | 1.9 | 2.3 | 2.8 | 3.2 | 3.5 |
| | Valency of acid | - | 1 | 1 | 1 | 1 | 1 | 1 | 1 | 1 |
| | Remaining amount of the original composition (mixed solution) (mL) | - | 500 | 480 | 460 | 440 | 420 | 400 | 380 | 360 |
| | Total amount of acid added (mEq/L) | - | 0.5 | 1.6 | 30 | 4.3 | 5.6 | 7.1 | 8.5 | 9.7 |
| Compositional change table (original composition: 500 mL + 1 ampoule (20 mL) of 1 meq/mL CaCl₂ correction solution) | Immediately after addition | × | × | × | × | ○ | ○ | ○ | ○ | ○ |
| | After 1 hour | × | × | × | × | ○ | ○ | ○ | ○ | ○ |
| | After 3 hours | × | × | × | × | ○ | ○ | ○ | ○ | ○ |
| | After 6 hours | × | × | × | × | × | ○ | ○ | ○ | ○ |
| | After 24 hours | × | × | × | × | × | × | × | ○ | ○ |

**[Table 14]**

| | | Unadjusted | Gluconic acid | | | | | | | |
|---|---|---|---|---|---|---|---|---|---|---|
| | Amount of acid added (amount added for that pH only) | 0 | (µL) | 300 | 300 | 230 | 290 | 250 | 250 | 250 |
| | | | 180 | | | | | | | |
| | pH of the mixed solution | 6.76 | 6.71 | 6.60 | 6.50 | 6.41 | 6.31 | 6.21 | 6.11 | 6.00 |
| | pH of the first-chamber infusion | 6.91 | 6.82 | 6.70 | 6.59 | 6.50 | 6.39 | 6.29 | 6.18 | 6.06 |

| | 3.15 mol/L | | | | | | | | | |
|---|---|---|---|---|---|---|---|---|---|---|
| Amount of acid added etc. | | Unadjusted | Glucon ic acid | | | | | | | |
| | pH of the mixed solution | 6.76 | 6.71 | 6.60 | 6.50 | 6.41 | 6.31 | 6.21 | 6.11 | 6.00 |
| | pH of the first-chamber infusion | 6.91 | 6.82 | 6.70 | 6.59 | 6.50 | 6.39 | 6.29 | 6.18 | 6.06 |
| | Total amount of acid added (mL) (taking into account the amount of acid removed together when 20 mL of the original composition is withdrawn each time) | - | 0.2 | 0.5 | 0.8 | 1.0 | 1.2 | 1.4 | 1.6 | 1.7 |
| | Total amount of acid added (meq) (taking into account the amount of acid removed together when 20 mL of the original composition is withdrawn each time) | - | 0.6 | 1.5 | 2.4 | 3.0 | 3.8 | 4.4 | 4.9 | 5.5 |
| | Valency of acid | - | 1 | 1 | 1 | 1 | 1 | 1 | 1 | 1 |
| | Remaining amount of the original composition (mixed solution) (mL) | - | 500 | 480 | 460 | 440 | 420 | 400 | 380 | 360 |
| | Total amount of acid added (mEq/L) | - | 1.1 | 3.1 | 5.2 | 6.8 | 9.0 | 10.9 | 13.0 | 15.2 |
| Compositional change table (original composition: 500 mL + 1 ampoule (20 mL) of 1 meq/mL CaCl₂ correction solution) | Immediately after addition | × | × | × | ○ | ○ | ○ | ○ | ○ | ○ |
| | After 1 hour | × | × | × | ○ | ○ | ○ | ○ | ○ | ○ |
| | After 3 hours | × | × | × | ○ | ○ | ○ | ○ | ○ | ○ |
| | After 6 hours | × | × | × | ○ | ○ | ○ | ○ | ○ | ○ |
| | After 24 hours | × | × | × | × | × | ○ | ○ | ○ | ○ |

**[Table 15]**

| | | Unadjusted | Lactic acid | | | | | | | |
|---|---|---|---|---|---|---|---|---|---|---|
| | Amount of acid added (amount added for that pH only) | 0 | (µL) | 60 | 60 | 50 | 45 | 40 | 40 | 40 |
| | | | 50 | | | | | | | |
| | pH of the mixed solution | 6.76 | 6.70 | 6.60 | 6.49 | 6.40 | 6.30 | 6.21 | 6.11 | 6.01 |
| | pH of the first-chamber infusion | 6.91 | 6.81 | 6.71 | 6.62 | 6.52 | 6.45 | 6.38 | 6.30 | 6.22 |

| | 11.8 mol/L | | | | | | | | | |
|---|---|---|---|---|---|---|---|---|---|---|
| Amount of acid added etc. | | Unadjusted | Lactic acid | | | | | | | |
| | pH of the mixed solution | 6.76 | 6.70 | 6.60 | 6.49 | 6.40 | 6.30 | 6.21 | 6.11 | 6.01 |
| | pH of the first-chamber infusion | 6.91 | 6.81 | 6.71 | 6.62 | 6.52 | 6.45 | 6.38 | 6.30 | 6.22 |
| | Total amount of acid added (mL) (taking into account the amount of acid removed together when 20 mL of the original composition is withdrawn each time) | - | 0.1 | 0.1 | 0.2 | 0.2 | 0.2 | 0.3 | 0.3 | 0.3 |
| | Total amount of acid added (meq) (taking into account the amount of acid removed together when 20 mL of the original composition is withdrawn each time) | - | 0.6 | 1.3 | 1.9 | 2.4 | 2.9 | 3.2 | 3.5 | 3.8 |
| | Valency of acid | - | 1 | 1 | 1 | 1 | 1 | 1 | 1 | 1 |
| | Remaining amount of the original composition (mixed solution) (mL) | - | 500 | 480 | 460 | 440 | 420 | 400 | 380 | 360 |
| | Total amount of acid added (mEq/L) | - | 1.2 | 2.7 | 4.2 | 5.5 | 6.8 | 8.0 | 9.2 | 10.5 |
| Compositional change table (original composition: 500 mL + 1 ampoule (20 mL) of 1 meq/mL CaCl₂ correction solution) | Immediately after addition | × | × | × | ○ | ○ | ○ | ○ | ○ | ○ |
| | After 1 hour | × | × | × | × | ○ | ○ | ○ | ○ | ○ |
| | After 3 hours | × | × | × | × | ○ | ○ | ○ | ○ | ○ |
| | After 6 hours | × | × | × | × | ○ | ○ | ○ | ○ | ○ |
| | After 24 hours | × | × | × | × | × | × | ○ | ○ | ○ |

**[Table 16]**

| | | Unadjusted | Citric acid | | | | | | | |
|---|---|---|---|---|---|---|---|---|---|---|
| | Amount of acid added (amount added for that pH only) | 0 | (µL) | 2000 | 2000 | 1700 | 2000 | 1800 | 1800 | 1700 |
| | | | 1000 | | | | | | | |
| | pH of the mixed solution | 6.76 | 6.71 | 6.61 | 6.50 | 6.41 | 6.31 | 6.21 | 6.11 | 6.01 |
| | pH of the first-chamber infusion | 6.91 | 6.85 | 6.73 | 6.62 | 6.53 | 6.42 | 6.33 | 6.23 | 6.14 |

| | 0.1 mol/L | | | | | | | | | |
|---|---|---|---|---|---|---|---|---|---|---|
| Amount of acid added etc. | | Unadjusted | Citric acid | | | | | | | |
| | pH of the mixed solution | 6.76 | 6.71 | 6.61 | 6.50 | 6.41 | 6.31 | 6.21 | 6.11 | 6.01 |
| | pH of the first-chamber infusion | 6.91 | 6.85 | 6.73 | 6.62 | 6.53 | 6.42 | 6.33 | 6.23 | 6.14 |
| | Total amount of acid added (mL) (taking into account the amount of acid removed together when 20 mL of the original composition is withdrawn each time) | - | 1.0 | 3.0 | 4.8 | 6.3 | 8.0 | 9.5 | 10.8 | 11.9 |
| | Total amount of acid added (meq) (taking into account the amount of acid removed together when 20 mL of the original composition is withdrawn each time) | - | 0.1 | 0.3 | 0.5 | 0.6 | 0.8 | 0.9 | 1.1 | 1.2 |
| | Valency of acid | - | 3 | 3 | 3 | 3 | 3 | 3 | 3 | 3 |
| | Remaining amount of the original composition (mixed solution) (mL) | - | 500 | 480 | 460 | 440 | 420 | 400 | 380 | 360 |
| | Total amount of acid added (meq/L) | - | 0.6 | 1.9 | 3.2 | 4.3 | 5.7 | 7.1 | 8.5 | 9.9 |
| Compositional change table (original composition: 500 mL + 1 ampoule (20 mL) of 1 meq/mL CaCl₂ correction solution) | Immediately after addition | × | × | × | ○ | ○ | ○ | ○ | ○ | ○ |
| | After 1 hour | × | × | × | × | ○ | ○ | ○ | ○ | ○ |
| | After 3 hours | × | × | × | × | ○ | ○ | ○ | ○ | ○ |
| | After 6 hours | × | × | × | × | ○ | ○ | ○ | ○ | ○ |
| | After 24 hours | × | × | × | × | × | ○ | ○ | ○ | ○ |

**[Table 17]**

| | | Unadjusted | Succinic acid | | | | | | | |
|---|---|---|---|---|---|---|---|---|---|---|
| | Amount of acid added (amount added for that pH only) | 0 | (µL) | 600 | 500 | 600 | 600 | 500 | 550 | 500 |
| | | | 100 | | | | | | | |
| | pH of the mixed solution | 6.76 | 6.71 | 6.60 | 6.51 | 6.40 | 6.30 | 6.21 | 6.11 | 6.01 |
| | pH of the first-chamber infusion | 6.91 | 6.90 | 6.78 | 6.69 | 6.57 | 6.46 | 6.36 | 6.26 | 6.15 |

| | 0.5 mol/L | | | | | | | | | |
|---|---|---|---|---|---|---|---|---|---|---|
| Amount of acid added etc. | | Unadjusted | Succinic acid | | | | | | | |
| | pH of the mixed solution | 6.76 | 6.71 | 6.60 | 6.51 | 6.40 | 6.30 | 6.21 | 6.11 | 6.01 |
| | pH of the first-chamber infusion | 6.91 | 6.90 | 6.78 | 6.69 | 6.57 | 6.46 | 6.36 | 6.26 | 6.15 |
| | Total amount of acid added (mL) (taking into account the amount of acid removed together when 20 mL of the original composition is withdrawn each time) | - | 0.1 | 0.7 | 1.2 | 1.7 | 2.2 | 2.6 | 3.1 | 3.4 |
| | Total amount of acid added (meq) (taking into account the amount of acid removed together when 20 mL of the original composition is withdrawn each time) | - | 0.1 | 0.3 | 0.6 | 0.9 | 1.1 | 1.3 | 1.5 | 1.7 |
| | Valency of acid | - | 2 | 2 | 2 | 2 | 2 | 2 | 2 | 2 |
| | Remaining amount of the original composition (mixed solution) (mL) | - | 500 | 480 | 460 | 440 | 420 | 400 | 380 | 360 |
| | Total amount of acid added (mEq/L) | - | 0.2 | 1.5 | 2.5 | 3.9 | 5.3 | 6.6 | 8.0 | 9.4 |
| Compositional change table (original composition: 500 mL + 1 ampoule (20 mL) of 1 meq/mL CaCl₂ correction solution) | Immediately after addition | × | × | × | × | ○ | ○ | ○ | ○ | ○ |
| | After 1 hour | × | × | × | × | ○ | ○ | ○ | ○ | ○ |
| | After 3 hours | × | × | × | × | × | ○ | ○ | ○ | ○ |
| | After 6 hours | × | × | × | × | × | ○ | ○ | ○ | ○ |
| | After 24 hours | × | × | × | × | × | × | ○ | ○ | ○ |

As shown in Tables 9 to 17, in cases in which the pH was adjusted with various organic acids, precipitation caused by a compositional change upon addition of a calcium chloride correction solution was suppressed for a specific period of time, as compared with cases in which the pH was adjusted with an inorganic acid. At the same pH, a greater effect of suppressing precipitation after a compositional change was observed generally in the following order, from the greatest to least: phosphoric acid, sulfuric acid, nitric acid, hydrochloric acid, acetic acid, succinic acid, lactic acid, and citric acid or gluconic acid (citric acid and gluconic acid showing substantially the same degree of effect).

## Claims

1. An infusion preparation for peripheral intravenous administration, comprising:
two chambers separated by a partition that is configured to allow fluid communication therebetween, the two chambers comprising:
a first chamber containing a first-chamber infusion containing an amino acid; and a second chamber containing a second-chamber infusion containing a sugar,
wherein a mixed solution of the first-chamber infusion and the second-chamber infusion:
(A) has a pH of 6.4 or less; and
(B) contains an organic acid in a total amount of 3 mEq/L or more.

2. The infusion preparation for peripheral intravenous administration according to claim 1, wherein the organic acid has a carboxy group.

3. The infusion preparation for peripheral intravenous administration according to claim 1 or 2, wherein the organic acid is a hydroxycarboxylic acid or acetic acid.

4. The infusion preparation for peripheral intravenous administration according to claim 1 or 2, wherein the mixed solution has a pH of 6.0 to 6.4.

5. The infusion preparation for peripheral intravenous administration according to claim 1 or 2, wherein the mixed solution contains at least one selected from the group consisting of citric acid, lactic acid, and acetic acid in a total amount of 55 mEq/L or more, or contains one or more other organic acids in a total amount of 3 mEq/L or more.

6. The infusion preparation for peripheral intravenous administration according to claim 1 or 2, wherein the organic acid is gluconic acid and/or citric acid.

7. The infusion preparation for peripheral intravenous administration according to claim 1 or 2, wherein the liquid obtained by removing the organic acid(s) in a total amount of 3 mEq/L or more from the mixed solution has a pH of 6.7 or more.

8. The infusion preparation for peripheral intravenous administration according to claim 1 or 2, wherein the pH of the first-chamber infusion is 6.55 or less.

9. A mixed solution of the first-chamber infusion and the second-chamber infusion according to claim 1 or 2, obtained by allowing fluid communication through the partition.

10. The infusion preparation for peripheral intravenous administration according to claim 1 or 2, for use in mixing the mixed solution with a calcium ion correction solution.

11. The infusion preparation for peripheral intravenous administration according to claim 10, wherein a ratio X/Y of a calcium ion content X (mEq/L) in the calcium ion correction solution to an organic acid content Y (mEq/L) in the mixed solution is 0.1 to 10.3.

12. A method for producing an infusion preparation for peripheral intravenous administration,
the infusion preparation for peripheral intravenous administration comprising two chambers separated by a partition that is configured to allow fluid communication therebetween, the two chambers comprising:
a first chamber containing a first-chamber infusion containing an amino acid; and
a second chamber containing a second-chamber infusion containing a sugar,
wherein a mixed solution of the first-chamber infusion and the second-chamber infusion:
(A) has a pH of 6.4 or less; and
(B) contains an organic acid in a total amount of 3 mEq/L or more,
the method comprising:
adding an organic acid in a total amount of 3 mEq/L or more to the first-chamber infusion and/or the second-chamber infusion, wherein, before the addition, the mixed solution of the first-chamber infusion and the second-chamber infusion has a pH of 6.7 or more, thereby changing a composition of the first-chamber infusion and/or the second-chamber infusion so that the pH of the mixed solution is 6.4 or less.
